# EUROPEAN PATENT SPECIFICATION

(11) **EP 2 277 042 B1**
(45) Date of publication and mention of the grant of the patent: **14.02.2018**
(21) Application number: 08793979.9
(22) Date of filing: 19.06.2008
(51) Int. Cl.: G01N 33/14, G01N 30/88, C07C 1/24

(54) **APPARATUS AND METHOD FOR DETERMINATION OF ISOTOPIC COMPOSITION OF NON-EXCHANGEABLE HYDROGEN AND DEUTERIUM ATOMS IN ETHANOL SAMPLES**
VORRICHTUNG UND VERFAHREN ZUR BESTIMMUNG DER ISOTOPENZUSAMMENSETZUNG NICHT AUSTAUSCHBARER WASSERSTOFF- UND DEUTERIUMATOME IN ETHANOLPROBEN
APPAREIL ET PROCÉDÉ PERMETTANT DE DÉTERMINER UNE COMPOSITION ISOTOPIQUE CONTENANT DES ATOMES D'HYDROGÈNE ET DE DEUTÉRIUM NON ÉCHANGEABLES DANS DES ÉCHANTILLONS D'ÉTHANOL

(30) Priority: 15.05.2008 RS 20080208
(43) Date of publication of application: 26.01.2011
(73) Proprietor: Smajlovic, Ivan, 21220 Becej (RS)
(72) Inventor: Smajlovic, Ivan, 21220 Becej (RS)
(86) International application number: PCT/RS2008/000022
(87) International publication number: WO 2009/139656

(56) References cited:
- WO-A-2007/055361
- US-A- 3 283 027
- US-A- 5 424 539
- SMITH G G ET AL: "A rapid method of qualitative and quantitative analysis of products from pyrolysis" ANALYST, vol. 86, July 1961 (1961-07), pages 480-483, XP002512893 ISSN: 0003-2654
- CALDERONE GIOVANNI ET AL: "Isotopic analysis of ethanol: study on O-18/O-16 measurement using a high-temperature pyrolysis system coupled to an isotope ratio mass spectrometer" RAPID COMMUNICATIONS IN MASS SPECTROMETRY, vol. 20, 2006, pages 937-940, XP002512894 ISSN: 0951-4198
- Keiko Ishida-Fujii ET AL: "Botanical and Geographical Origin Identification of Industrial Ethanol by Stable Isotope Analyses of C, H, and O", BIOSCIENCE BIOTECHNOLOGY BIOCHEMISTRY., vol. 69, no. 11, 1 January 2005 (2005-01-01), pages 2193-2199, XP55291673, TOKYO, JAPAN ISSN: 0916-8451, DOI: 10.1271/bbb.69.2193
- KELLY SIMON D ET AL: "Emerging techniques in vegetable oil analysis using stable isotope ratio mass spectrometry", GRASAS Y ACEITES, CONSEJO SUPERIOR DE INVESTIGACIONES CIENTIFICAS (SPANISH NATIONAL RESEARCH COUNCIL), SPAIN, vol. 53, no. 1, 1 January 2002 (2002-01-01), pages 34-44, XP009100117, ISSN: 0017-3495, DOI: 10.3989/GYA.2002.V53.I1.288
- John Dunbar ET AL: "Bestimmung der 2H/1H-Verh�ltnisse kohlenstoffgebundener Wasserstoffatome in Zuckern", FRESENIUS ZEITSCHRIFT FUER ANALYTISCHE CHEMIE., vol. 317, no. 8, 1 January 1981 (1981-01-01), pages 853-857, XP55291696, DE ISSN: 0016-1152, DOI: 10.1007/BF00466937

## Description

### Technical Field

The present invention is related to apparatus and method for online determination of isotopic composition of non-exchangeable hydrogen and deuterium atoms in ethanol samples for the purpose of authenticity and geographical origin determination of wines and grape musts, beer, strong spirits, fruit juices, honeys and all other food products which contains alcohol (ethanol) and/or fermentable sugars.

### Background art

Isotopic methods have shown that they can be very powerful analytical tool for authenticity and geographical origin determination of wines and strong spirits. By measuring the content of stable isotopes in these products useful information can be provided for detection of many frauds in wine and strong spirits production. Instrumental techniques which are used for isotopic measurements are based on measuring the relative ratios of stable isotopes by means of Isotope Ratio Mass Spectrometry.

Systems comprising a pyrolysis chamber and continuous flow isotope spectrometer CF-TC/EA - IRMS (Flash HT Continuous Flow - Temperature Conversion/Elemental Analyzer - Isotope Ratio Mass Spectrometry from Thermo Electron Corporation) are commercially available for stable hydrogen analysis of solid and liquid samples.

BIOSCIENCE, BIOTECHNOLOGY, BIOCHEMISTRY, vol. 69, -no. 11, pages 2193 - 2199, 2005; XP055291673 describes the determination of the isotope ratios of carbon, hydrogen and oxygen of rectified alcohols to distinguish their botanical and geographical origins by continuous flow - isotope ratio mass spectrometry.

GRASAS Y ACEITAS, VOL.53, NO. 1, PAGES 34-44, 2002; XP009100117 reviews the application of stable isotope ratio mass spectrometry to authenticating fruit products, especially vegetable oil. When analyzing ethanol samples, by means of CF - TC/EA - IRMS (Continuous Flow - Thermal Conversion/Elemental Analyzer - Isotope Ratio Mass Spectrometry), because of the ethanol's hydroxyl group, which contains easily exchangeable hydrogen, gained δD values for ethanol of the same botanical and geographical origin can vary, and for that reason it is impossible to perform qualitative and quantitative identification of the ethanol sample origin.

One of the problems which can occur, for example, in strong spirit production, is in finalization production steps. The distillate is diluted with water to determined alcoholic strength which is necessary so that alcoholic drink could be consumed. By adding water which has different isotopic content, dynamic isotopic equilibrium is disturbed and hydrogen or deuterium which is bonded to oxygen atom of the hydroxyl group is exchanged. This is one of the reasons for gaining the wrong δD values and wrong information about ethanol origin.

One approach to the problem of exchangeable hydrogens in the context of stable isotope mass spectrometry as applied to detecting D/H ratios in sugars is discussed in FRESENIUS ZEITSCHRIFT FÜR ANALYTISCHE CHEMIE, vol. 517, no. 8, pages 853 - 857, 1981; XP055291696, where the problem is solved by forming nitrate derivatives.

Because of the problems which are stated above, instrumental technique CF - TC/EA- IRMS can not be very useful in detection of frauds in wine and alcoholic drinks production and moreover for detection of ethanol which originates from beet sugar, barley, wheat etc. in wine and alcoholic drinks. Accordingly, in this invention, an alcohol thermal dehydration chamber, gives the possibility to dehydrate ethanol and to remove exchangeable hydrogen (or deuterium) atom of the hydroxyl group without any lost or isotopic fractionation. In this way stabilization and constant δD values are achieved and this comes from other atoms of hydrogen and deuterium which are strongly bonded to carbon atoms inside of ethene (ethylene) gas which is prepared from ethanol sample with particular botanical origin.

Dehydration reactors are known from US 3 283 027 A, THE ANALYST, vol. 86, pages 480 - 483, 1961; XP002512893 and WO 2007/055361 A.

### Disclosure of the Invention

Deuterium and hydrogen relative ratio measurements for the proposal of authenticity and origin determination of wines and alcoholic drinks, beer, fruit juices and honey today is done by means of SNIF -NMR (Site Natural Isotopic Fractionation - Nuclear Magnetic Resonance) which is based on intermolecular scanning of the measured ethanol sample and determination of isotopic composition of hydrogen and deuterium atoms sited on the first and on the second carbon atom inside of the ethanol molecule. Results gained by SNIF - NMR method give the information about the presence of ethanol which originates from beet sugar or other industrial plants, and which belongs to the C3 plant group.

This instrumental technique is very precise, but it has some lacks. Some of those lacks are:
- In the first place SNIF - NMR is very expansive instrument and method and it requires big financial funds (big consumption of helium and liquid nitrogen and also electrical energy),
- Time needed for the analysis is quite long (small number of samples can be analyzed),
- It occupies big part of the working place because of it's size and because of the very strong magnetic field which it makes (security zone is needed),
- Standards which are used in analysis are expensive.

### Disclosure of the Invention

The main goal of this invention is to overcome the barrier and lacks of today known apparatus and methods for determination of isotopic composition of non-exchangeable hydrogen and deuterium atoms in ethanol samples.

The foregoing and further objects are achieved by the apparatus for online determination of isotopic composition of non-exchangeable hydrogen and deuterium atoms in ethanol samples of claim 1 and the method for online determination of isotopic composition of non-exchangeable hydrogen and deuterium atoms in ethanol samples of claim 4. For use in this invention the alcohol thermal dehydration chamber comprises of: the reaction vessel which contains upper opening with the stopper and septa, which is used for the ethanol sample injection by syringe, and sideway left opening with the valve which which which purpose is purging of the chamber with the inert gas helium. One part of the reaction vessel poses thermo-jacket which comprises of two electrical heaters. Also as the alternative for thermo-jacket the open flame can be used for the reaction vessel heating. The second part of the reaction vessel, this part has a tube shape turned to the right side, contains dehydration catalyst and for that purpose aluminum oxide (Al₂O₃), silica gel, zeolite or the mixture of those substances or similar materials can be used. At the right end the reaction vessel is connected with tube with the gas tight stopper. Inside of this tube with a gas tight stopper small amount of silica gel or similar inert hydroscopic material can be placed. The right end of this tube with gas tight stopper contains two valves, which are used for purging of the alcohol thermal dehydration chamber with the helium gas. The alcohol thermal dehydration chamber lies on the stand.

The alcohol thermal dehydration chamber is applied as the part of the apparatus for online determination of isotopic composition of non-exchangeable hydrogen and deuterium atoms in ethanol samples. It can also be used for the offline preparation of ethene (ethylene) samples. If it is used in this way, the alcohol thermal dehydration chamber is connected to the previously vacuumed vial which has the role for taking the ethene gas. Prepared ethene gas is then injected with the gas tight syringe on to existing state of instrumental technique CF - TC/EA - IRMS or GC/TC - IRMS (Gas Chromatography/Thermal Conversion - Isotope Ratio Mass Spectrometer).

According to this invention the apparatus for online determination of isotopic composition of non-exchangeable hydrogen and deuterium atoms in ethanol samples comprises of: A) alcohol thermal dehydration chamber, B) detection device, which contains pyrolysis reactor and the continuous flow isotope spectrometer, and it is connected to the alcohol thermal dehydration chamber over C) system of valves, connectors and capillary tubes which are used for transfer of analyzed sample as for the purging of the alcohol thermal dehydration chamber.

It will be understood that the apparatus will generally comprise additional features known in the art e.g. control and processor units interacting with the functional components of the apparatus (automatic manipulating). It may be contemplated to adopt or appropriately modify available laboratory equipment to control and interact with the apparatus of this invention.

According to this invention the procedure and principle for online determination of isotopic composition of non-exchangeable hydrogen and deuterium atoms in ethanol samples is conceived on preliminary purging of the alcohol thermal dehydration chamber in stream of inert gas helium, injection of the ethanol sample into the reaction vessel (injection can be done manually or by means of auto-sampler), alcohol heating and production of overheated alcoholic fumes, and crossing of those ethanol fumes over dehydration catalyst (aluminum oxide - Al₂O₃, silica gel, zeolite or the mixture of those substances or similar materials), in the stream of the carrier gas helium to the pyrolysis reactor. Elemental gases produced by pyrolysis of prepared ethene (H₂ and CO) goes through the gas chromatography column where are separated, and over interface and the Open Split enter IRMS where are detected. Offline preparation of ethene samples using the alcohol thermal dehydration chamber comprises preliminary purging of the alcohol thermal dehydration chamber in stream of the inert gas helium through the left sideway by opening the valve for the helium gas. Before purging starts it is needed to open valves on the right side of the chamber. After purging is done it is needed to close right sided valves and then the valve for the helium gas. After purging on the metal needle, at the right end, connect the previously vacuumed vial, and then open the valve on the right side of the chamber. Further, by heating the reaction vessel by means of thermo jacket or alternatively by open flame, and injecting the ethanol sample to the reaction vessel, dehydration reaction takes place and formed gas ethene is captured inside the vial. After this vial is taken off from the chamber and ethene is then manually injected with the gas tight syringe on to existing state of instrumental techniques CF - TC/EA - IRMS or GC/TC - IRMS.

### Brief Description of Drawings

The above mentioned and other features and objects of this invention and the manner of achieving them will become more apparent and this invention itself will be better understood by reference to the following description of various embodiments of this invention taken in conjunction with the accompanying drawings, wherein:
Fig. 1 - Shows review of the alcohol thermal dehydration chamber;
Fig. 2 - Shows review of the apparatus for online determination of isotopic composition of non-exchangeable hydrogen and deuterium atoms in ethanol samples;
Fig. 3 - Shows an alcohol thermal dehydration chamber for offline preparation of ethene samples and for the existing state of instrumental techniques CF - TC/EA - IRMS or GC/TC - IRMS

### Best Modes for Carrying Out of the Invention

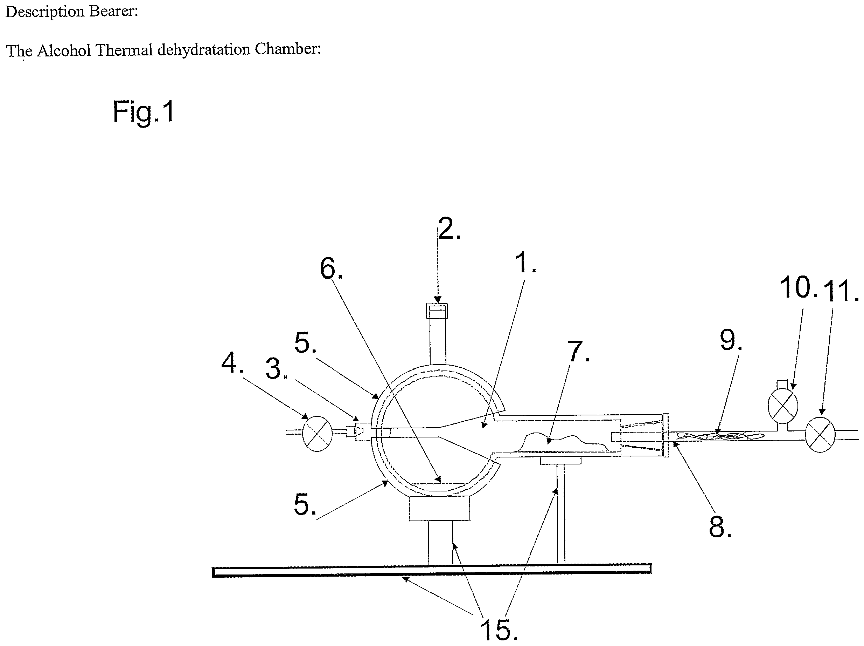

In accordance with the idea of the invention figures 1, 2 and 3 are showing pictures of the alcohol thermal dehydration chamber, apparatus for online determination of isotopic composition of non-exchangeable hydrogen and deuterium atoms in ethanol samples and variant solution of the alcohol thermal dehydration chamber for offline preparation for ethene (ethylene) samples for the existing state of instrumental techniques CF - TC/EA - IRMS or GC/TC - IRMS. Fig. 1 shows the alcohol thermal dehydration chamber. Fig.1 shows all important parts of the alcohol thermal dehydration chamber. The alcohol thermal dehydration chamber comprises of: the reaction vessel (1) which contains upper opening with the stopper and septa (2), which is used for injection of ethanol sample (6) by syringe, and sideway left opening (3) with the valve (4) which purpose is purging of the chamber with the inert gas helium. One part of the reaction vessel poses thermo-jacket (5) which comprises of two electrical heaters. Also as the alternative to thermo-jacket (5) the open flame can be used for the reaction vessel heating. The second part of the reaction vessel, this part has a tube shape turned to the right side at fig.1, contains dehydration catalyst (7) and for that purpose aluminum oxide (Al₂O₃), silica gel, zeolite or the mixture of those substances or similar materials can be used. At the right end the reaction vessel is connected with tube with the gas tight stopper (8). Inside of this tube with the gas tight stopper (8) small amount of silica gel or similar inert hydroscopic material can be placed (9). The right end of this tube with the gas tight stopper (8) contains two vents (10) and (11), which are used for purging of the alcohol thermal dehydration chamber with the helium gas. The alcohol thermal dehydration chamber lies on the stand (15).

In accordance with the idea of this invention the alcohol thermal dehydration chamber gives the possibility to dehydrate ethanol and to remove exchangeable hydrogen (or deuterium) atom of the hydroxyl group without any lost or isotopic fractionation. In that way stabilization and constant δD values are achieved and this comes from other hydrogen and deuterium atoms which are strongly bonded to carbon atoms inside of ethene (ethylene) gas which is prepared from ethanol sample with particular botanical origin.

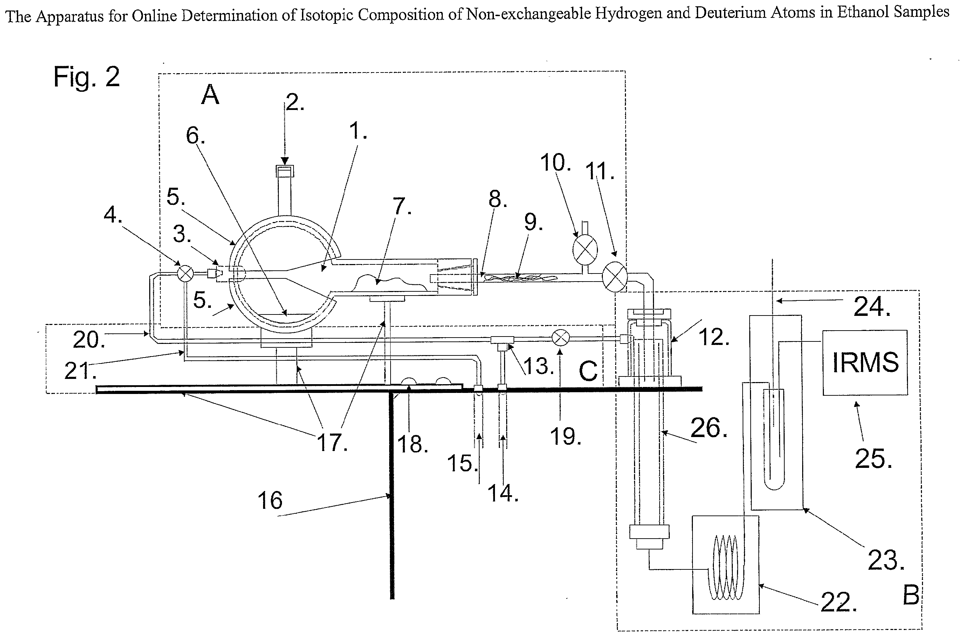

In accordance with the idea of this invention fig.2 shows in detail the apparatus for the online determination of isotopic composition of non-exchangeable hydrogen and deuterium atoms in ethanol samples. Fig.2 shows all important parts of the apparatus for the online determination of isotopic composition of non-exchangeable hydrogen and deuterium atoms in ethanol samples from which it is comprised.

In accordance with the idea of this invention the apparatus for online determination of isotopic composition of non-exchangeable hydrogen and deuterium atoms in ethanol samples comprises of: A) alcohol thermal dehydration chamber, B) detection device, which contains pyrolysis reactor and the continuous flow isotope spectrometer, and it is connected to the alcohol thermal dehydratation chamber over C) system of valves, connectors and capillary tubes which are used for transfer of analyzed sample as for the purging of the alcohol thermal dehydratation chamber. The alcohol thermal dehydration chamber, which is the part of the apparatus for online determination of isotopic composition of non-exchangeable hydrogen and deuterium atoms in ethanol samples, comprises of: the reaction vessel (1) which contains upper opening with stopper and septa (2), which is used for injection of ethanol sample (6) by syringe, and left sideway opening (3) with the "Switch" valve for the "Reference" and "Carrier" gas helium (4). One part of the reaction vessel poses thermo-jacket (5) which comprises of two electrical heaters. The second part of the reaction vessel, this part has a tube shape turned to the right side at fig.2, contains dehydration catalyst(7) and for that purpose aluminum oxide (Al₂O₃), silica gel, zeolite or the mixture of those substances or similar materials can be used. At the right end the reaction vessel is connected with tube with the gas tight stopper (8). Inside of this tube with the gas tight stopper (8) small amount of silica gel or similar inert hydroscopic material can be placed (9). The right end of this tube with the gas tight stopper (8) contains two valves (10) and (11). Tube with the gas tight stopper (8) at its end is connected via injection connector (12) directly to the pyrolysis reactor (26), which is connected via gas chromatography column (22) and Interface Open Split (23), which has the capillary for Helium dilution (24), with the isotopic mass spectrometer IRMS (25). The alcohol thermal dehydration chamber lies on the stand (17) which is fixed to the housing of the peripheral (16), which contains pyrolysis reactor (26), with screws (18). Capillary tube (21) at one end is connected with the output of the helium "Reference" gas (15), which is used for chamber purging, and at it's other end connected with the "Switch" valve (4). The capillary tube (20) is, over "T" connector (13), at one end, connected with the main flow output of the helium "Carrier" gas (14), and at it's other end connected with the "Switch" valve (4). The main flow output of the helium "Carrier" gas (14) is connected via "T" connector (13) and the capillary tubing with the security valve (19) and injection connector (12) on the pyrolysis reactor (26).

In accordance with the idea of this invention the procedure and principle for working with the apparatus for online determination of isotopic composition of non-exchangeable hydrogen and deuterium atoms in ethanol samples follows as is:

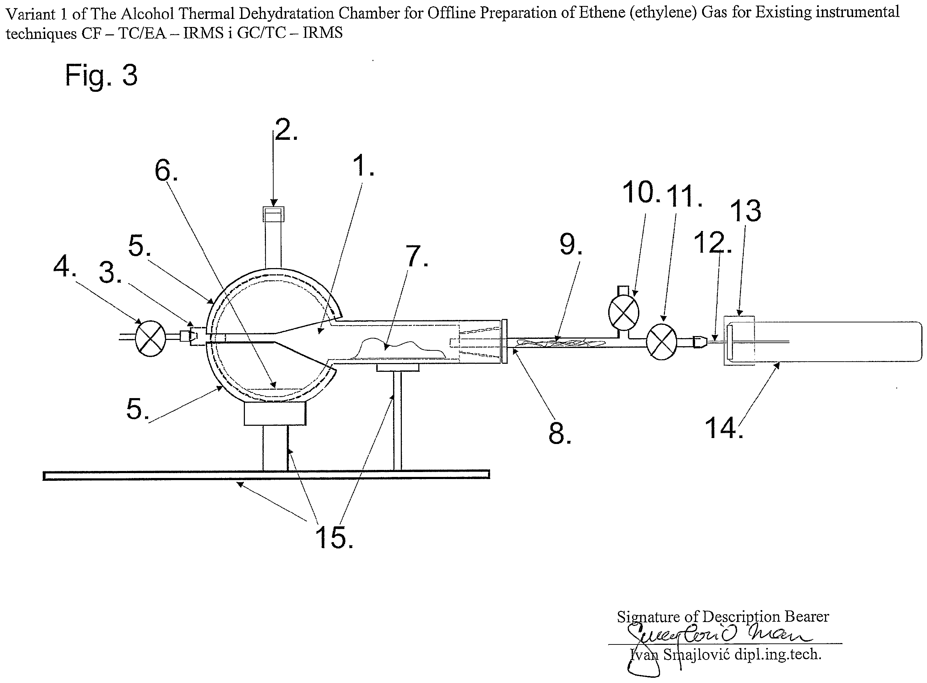

The first phase is related to purging of the alcohol thermal dehydration chamber, which is the part of the apparatus for online determination of isotopic composition of non-exchangeable hydrogen and deuterium atoms in ethanol samples, with the inert gas helium. Before purging begins it is necessary to close the valve (11) and to open the vent (10), and then, over sideway opening (3) and by moving the "Switch" valve (4) to the position for the "Reference" gas helium (21) start to purge the alcohol thermal dehydration chamber. The flow of the "Reference" gas helium should be in the range from 20 ml/min. to 200 ml/min. After purging is done, this should last maximum 15 minutes, close the vent (10), and then open the valve (11) and move the "Switch" valve (4) in the position for the main flow of the "Carrier" gas helium (20). The flow of the "Carrier" gas helium should be in the range from 70ml/min. to 170ml/min. After purging of the alcohol thermal dehydration chamber is done, heating of the reaction vessel (1) by means of the thermo-jacket (5) may start until the temperature reaches the range from 250°C to 500°C. After this is done inject maximum 1ml, of preliminary distillated and isolated alcohol (ethanol) sample from analyzed wine, beer or alcoholic drink or similar. By entering the reaction vessel, sample is momentarily vaporized into overheated alcoholic fume which, in the stream of helium, passes over dehydration catalyst (7). With dehydration and separation of water and absorption by the catalyst in the surplus, prepared ethene (ethylene) gas, through the capillary tube with the gas tight stopper (8) and opened valve (11), is entering the pyrolysis reactor (26) and then, over gas chromatography column (22) and Interface with the Open Split (23) is detected on the isotope mass spectrometer IRMS (25). Formed gas ethene (ethylene) prepared by means of the alcohol thermal dehydration is degraded by pyrolysis to the elemental gases (H₂ and CO) which then pass through the gas chromatography column where are separated, and then over Interface and its Open Split are entering the IRMS where are finally detected. Fig. 3 shows an alcohol thermal dehydration chamber for offline preparation of ethene (ethylene) samples for the existing state of instrumental techniques CF-TC/EA-IRMS or GC/TC - IRMS. Fig.3 shows an alcohol thermal dehydration chamber for offline preparation of ethene (ethylene) samples for the existing state of instrumental techniques CF-TC/EA-IRMS or GC/TC-IRMS. The alcohol thermal dehydration chamber comprises of:
reaction vessel (1) which contains upper opening with the stopper and septa (2), which is used for injection of ethanol sample (6) by syringe and sideway left opening (3) with the valve (4) which purpose is purging of the chamber with the inert gas helium. One part of the reaction vessel poses thermo-jacket (5) which comprises of two electrical heaters. Also as the alternative for the thermo-jacket (5) the open flame can be used for the reaction vessel heating. The second part of the reaction vessel, this part has a tube shape turned to the right side at fig. 1, contains dehydration catalyst (7)
and for that purpose aluminum oxide (Al₂O₃), silica gel, zeolite or the mixture of those substances or similar materials can be used. At the right end the reaction vessel is connected with tube with the gas tight stopper (8). Inside of this tube with a gas tight stopper (8) small amount of silica gel or similar inert hydroscopic material can be placed (9). The right end of this tube with the gas tight stopper (8) contains two valves (10) and (11), which are used for purging of the alcohol thermal dehydration chamber with the helium gas. At the right end, the tube with the gas tight stopper (8) is connected with the metal needle (12) and via stopper (13) with the vial (14). The alcohol thermal dehydration chamber lies on the stand (15).

In accordance with the idea of this invention the procedure for the offline preparation of ethene (ethylene) samples by means of the alcohol thermal dehydration chamber follows as is:
The first phase is related with purging of the alcohol thermal dehydration chamber with the inert gas helium over the sideway opening (3) by opening the valve for the helium gas (4). Before releasing the helium stream it is needed to open the vents (10) and (11). After purging of the dehydration chamber the valves (10) and (11) have to be closed and then the valve for the helium gas (4) is closed too. After this is done, preliminary vacuumed vial (14) is put on the metal needle (13) and then the valve (11) is opened. After purging of the alcohol thermal dehydration chamber is done, heating of the reaction vessel (1) by means of the thermo-jacket (5) may start until the temperature reaches the range from 250°C to 500°C. Alternatively for heating of the reaction vessel open flame can be used. After this is done preliminary distillated and isolated alcohol (ethanol) sample from analyzed wine, beer or alcoholic drink or similar is injected and after reaction time of maximum 5 minutes the valve (11) is closed and the vial (14) is disconnected from the dehydratation chamber. Prepared gas ethene (ethylene) caught inside the vial (14) is used for manual injection by means of the gas tight injection syringe to the instrumental system CF - TC/EA - IRMS or GC/TC - IRMS.

In accordance with the idea of this invention, the apparatus for thermal alcohol dehydration and the procedure for determination of relative isotopic composition of all non-exchangeable hydrogen and deuterium atoms in ethanol samples, and for the purpose of authenticity and geographical origin determination of wines and grape musts, beers, alcoholic drinks, fruit juices, honey and all other food products which contain alcohol and/or fermentable sugars, it has the following advantages:
- In the first place, it gives very good precision and repeatability of results for δD values of analyzed ethanol samples, no matter if ethanol sample was diluted with water before distillation, and it gives espied constant difference and dependence between ethanol samples with botanical origin from C3 group of plants;
- Time of the analysis is quit shorter in compare to the SNIF - NMR;
- There is no need for big financial funds and special conditions for maintenance like which is the case with instrumental technique SNIF-NMR,
- No safety zone is needed,
- It gives the opportunity to detect the presence of ethanol which originates from beet sugar, wheat, barley and other industrial plants which belong to the C3 group of plants, in the ethanol samples which are isolated from the analyzed wines and alcoholic drinks or fermented juices and fermented honey.
It will be appreciated that modifications to the embodiments described above are of course possible. Accordingly the present innovation is not limited to the embodiments described above.

### Industrial Applicability

The apparatus and procedure for online determination of isotopic composition of non-exchangeable hydrogen and deuterium atoms in ethanol samples are applicable in instrumental analytical chemistry and are used for authenticity and geographical origin determination of wines and grape musts, alcoholic drinks, beers, fruit juices, honey and other food products which contain ethanol and/or fermentable sugars.

## Claims

1. Apparatus for the online determination of isotopic composition of non-exchangeable hydrogen and deuterium atoms in ethanol samples comprising:
- an alcohol dehydration chamber (1) for converting the ethanol sample into ethene comprising means for purging the chamber; means for injecting a sample (2); heating means (5); a dehydration catalyst (7); and means for supplying carrier gas;
- a pyrolysis reactor (26) for degrading said ethene gas into a gaseous mixture comprising hydrogen and carbon monoxide gases;
- a gas chromatography column (22) for separating hydrogen from the gaseous mixture;
- an open split interface (23); and
- an isotopic ratio mass spectrometer IRMS (25) for determining the isotopic composition of said hydrogen gas.

2. Apparatus according to claim 1, wherein the dehydration catalyst comprises aluminium oxide (Al₂O₃), silica gel, zeolite or a mixture thereof.

3. Apparatus according to claim 1 or 2, further comprising a hygroscopic material (9) for removing water molecules created by the conversion of the ethanol sample into ethene in the alcohol dehydration chamber.

4. Method for the online determination of isotopic composition of non-exchangeable hydrogen and deuterium atoms in ethanol samples, using the apparatus of claims 1-3, comprising the steps of:
- purging the alcohol dehydration chamber with an inert gas;
- injecting the ethanol sample into the alcohol dehydration chamber;
- heating the chamber thereby vaporising the ethanol sample producing fumes which pass over the dehydration catalyst, dehydrating ethanol by removing exchangeable hydrogen and forming ethene;
- passing said ethene in a carrier gas to the pyrolysis reactor (26) where ethene is degraded into elemental gases hydrogen and carbon monoxide;
- separating hydrogen from the gaseous mixture in the gas chromatography column (22);
- transferring said hydrogen gas via open split interface (23) to the isotopic ratio mass spectrometer IRMS (26); and
- measuring the isotopic ratio of said hydrogen gas.

5. Method of claim 4 further comprising comparing the measured hydrogen to deuterium relative ratio with the relative ratio of the hydrogen to deuterium in ethene obtained from ethanol samples of known origin, thereby determining authenticity and geographical origin of the sample.

6. Method of claim 4 or 5, wherein the ethanol sample is selected from wine and great musts, alcoholic drinks, beers, fruit juices, honey and other food products, which contain ethanol and/or fermentable sugars.

## Patentansprüche

1. Apparatur für die Online-Bestimmung der Isotoperizueammensetzung von nicht austauschbaren Wasserstoff- und Deuteriumatomen in Ethanolproben, umfassend:
- eine Alkoholdehydratationskammer (1) zur Umwandlung der Ethanolprobe in Ethen, umfassend Mittel zum Spülen der Kammer; ein Mittel zum Einspritzen einer Probe (2); ein Heizmittel (5); einen Dehydratationskatalysator (7); und ein Mittel für die Lieferung von Trägergas:
- eine Pyrolyse, die das Ethengas zu einem gasförmigen Gemisch aus Wasserstoff und Kohlenmonoxidgasen degradiert;
- eine Gaschromatographie-Säule (22) für die Heraustrennung von Wasserstoff aus der gasförmigen Mischung;
- eine offene Teilungsschnittstelle (23); und - ein Isotopenverhältnis-Massenspektrometer IRMS (25) zur Bestimmung der Isotopenzusammensetzung des Wasserstoffgases.

2. Vorrichtung nach Anspruch 1, wobei der Dehydratisierungskatalysator Aluminiumoxid (Al₂O₃), Kieselgel, Zeolith, oder ein Gemisch davon, umfasst.

3. Vorrichtung nach Anspruch 1 oder 2, ferner umfassend ein hygroskopisches Material (9) für die Beseitigung von Wassermolekülen, die durch die Umwandlung der Ethanolprobe in Ethen in der Alkoholdehydratationskammer erzeugt wurden.

4. Verfahren zur Online-Bestimmung der Isotopanzusammensetzung von nicht austauschbaren Wasserstoff- und Deuteriumatomen in Ethanolproben, unter Verwendung der Vorrichtung nach den Ansprüchen 1-3, umfassend die Schritte:
- Spülen der Alkohol-Dehydratationskammer mit einem Inertgas;
- Injektion der Ethanolprobe in die Alkoholdehydratationskammer;
- Erhitzen der Kammer, wodurch die Ethanolproben, die Dämpfe produzieren, verdampft werden, die den Dehydratationskatalysator passieren, wobei das Ethanol durch Entfernen von austauschbarem Wasserstoff und die Bildung von Ethen dehydratisiert wird;
- Übergabe des Ethen in einem Trägergas zur Pyrolyse (26), wo das Ethen zu den elementaren Gasen Wasserstoff und Kohlenmonoxid degradiert wird;
- Abscheidung von Wasserstoff aus dem gasförmigen Gemisch in der Gaschromatographiesäule (22);
- Übertragung des Wasserstoffgases über eine offene Teilungsschnittstelle (23) zu einem Isotopenverhältnis-Massenspektrometer IRMS (26); und
- Messen des Isotopenverhältnisses des genannten Wasserstoffgases.

5. Verfahren nach Anspruch 4, **dadurch gekennzeichnet, dass** man das gemessene relative Wasserstoff/Deuterium-Verhältnis mit dem relativen Wasserstoff/Deuterium-Verhältnis in Ethen, erhaltenen Ethanolproben bekannten Ursprungs, vergleicht, wodurch die Echtheit und der geographische Ursprung der Probe bestimmt werden.

6. Verfahren nach Anspruch 4 bis 5, wobei die Ethanolprobe aus Wein und Most, alkoholischen Getränken, Früchten, Honig und anderen Nahrungsmittelprodukten ausgewählt worden ist, die Ethanol und/oder fermentierbare Zucker enthalten.

## Revendications

1. L'appareil pour détermination en ligne de la composition isotopique des atomes d'hydrogène et de deutérium non échangeables dans un échantillon d'éthanol, comprenant:
- une chambre de déshydratation d'alcool (1) pour la conversion de l'échantillon d'éthanol en éthène, équipée d'un dispositif de purge de la chambre, dispositif d'injection de l'échantillon (2), dispositif de chauffage (5), d'un catalyseur de déshydratation (7), et des moyens de fourniture d'un gaz porteur;
- un réacteur de pyrolyse (26) pour la dégradation de l'éthène suscité en un mélange gazeux contenant de l'hydrogène et du monoxyde de carbone gazeux;
- une colonne de chromatographie en phase gazeuse (22) pour la séparation de l'hydrogène du mélange gazeux;
- une interface ouverte divisée (23); et - un spectromètre de masse de rapport isotopique IRMS (25) pour la déterminer la composition isotopique du gaz hydrogène suscité.

2. L'appareil selon la revendication 1, dans lequel le catalyseur de déshydratation contient de l'oxyde d'aluminium (Al₂O₃), du gel de silice, de la zéolite ou un mélange de ces substances.

3. L'appareil selon la revendication 1 ou 2, contentant en plus un matériau hygroscopique (9) pour supprimer des molécules d'eau créées lors de la conversion de l'échantillon d'éthanol en éthène dans la chambre de déshydratation d'alcool.

4. Méthode de détermination en ligne de la composition isotopique des atomes d'hydrogène et de deutérium non échangeables dans des échantillons d'éthanol, en utilisant l'appareil selon les revendications 1 - 3, comprenant les étapes suivantes:
- purge de la chambre de déshydratation d'alcool avec un gaz inerte;
- injection de l'échantillon d'éthanol dans la chambre de déshydratation d'alcool;
- chauffage de la chambre de déshydratation d'alcool causant la vaporisation de l'échantillon d'éthanol et produisant des émanations qui passent au-dessus du catalyseur de déshydratation, où s'effectue la déshydratation de l'éthanol en éliminant de l'hydrogène échangeable et en formant de l'éthène;
- passage dudit éthène en un gaz porteur jusqu'au réacteur de pyrolyse (26) où l'éthène est dégradé en hydrogène et monoxyde de carbone élémentaires;
- séparation de l'hydrogène du mélange gazeux dans la colonne de chromatographie en phase gazeuse (22);
- transfert dudit hydrogène gazeux par une interface ouverte divisée (23) jusqu'au spectromètre de masse de rapport isotopique IRMS (25); et
- mesure du rapport isotopique dudit hydrogène gazeux.

5. Méthode selon la revendication 4, comprenant la comparaison entre le rapport relatif mesuré entre hydrogène et deutérium dans l'éthène obtenu des échantillons d'éthanol d'origine connue, déterminant ainsi l'authenticité et l'origine géographique de l'échantillon.

6. Méthode selon la revendication 4 ou 5, **caractérisée en ce que** l'échantillon d'éthanol est extrait du vin et du moût, des boissons alcoolisées, des bières, des jus de fruits, du miel et d'autres produits alimentaires qui contiennent de l'éthanol et/ou des sucres fermentables.
